(19) Europäisches Patentamt
European Patent Office
Office européen des brevets

(11) Veröffentlichungsnummer: **0 156 309**
**B1**

(12) # EUROPÄISCHE PATENTSCHRIFT

(45) Veröffentlichungstag der Patentschrift: **14.06.89**

(51) Int. Cl.⁴: **C 07 C 53/02**, C 07 C 51/09, C 07 C 51/44, C 07 C 51/48

(21) Anmeldenummer: **85103303.5**

(22) Anmeldetag: **21.03.85**

(54) **Verfahren zur Gewinnung von wasserfreier oder weitgehend wasserfreier Ameisensäure durch Hydrolyse von Methylformiat.**

(30) Priorität: **28.03.84 DE 3411384**

(43) Veröffentlichungstag der Anmeldung: **02.10.85 Patentblatt 85/40**

(45) Bekanntmachung des Hinweises auf die Patenterteilung: **14.06.89 Patentblatt 89/24**

(84) Benannte Vertragsstaaten: **BE DE FR GB IT NL**

(56) Entgegenhaltungen:
**EP-A- 0 012 321**
**EP-A- 0 017 866**
**DE-A- 2 545 658**

(73) Patentinhaber: **BASF Aktiengesellschaft, Carl-Bosch-Strasse 38, D-6700 Ludwigshafen (DE)**

(72) Erfinder: **Bott, Kaspar, Dr., Rieslingweg 4, D-6706 Wachenheim (DE)**
Erfinder: **Kaibel, Gerd, Dipl. Ing., Robert-Bosch-Strasse 4, D-6840 Lampertheim 1 (DE)**
Erfinder: **Mittelstaedt, Erich, Dipl.-Ing., Langgasse 10, D-6501 Bodenheim (DE)**
Erfinder: **Imich, Rudolf, Dr., In den Hahndornen 2, D-6719 Bobenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 156 309 B1**

ACTORUM AG

## Beschreibung

Die vorliegende Erfindung betrifft ein Verfahren zur Gewinnung von wasserfreier oder weitgehend wasserfreier Ameisensäure durch Hydrolyse von Methylformiat unter Einsatz von Extraktionsmitteln, bestehend aus Carbonsäureamiden der allgemeinen Formel I

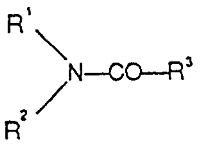

(I)

wobei $R^1$ und $R^2$ Alkylgruppen, die auch zu einem 5- oder 6gliedrigen Ring verbunden sein können, oder $R^1$ Wasserstoff und $R^3$ Wasserstoff oder einen $C_1$-$C_4$-Alkylrest bedeuten, und die Summe der C-Atome in den Resten $R^1$-$R^3$ 1 bis 14 beträgt.

Die Gewinnung von wasserfreier Ameisensäure durch Hydrolyse von Methylformiat ist in der Patentliteratur mehrfach beschrieben, beispielsweise in den DE-OS 2 744 313, 2 853 991 und 2 914 671.

So ist aus der DE-OS 2 744 313 bekannt, die Hydrolyse des Methylformiats in Gegenwart einer organischen Base, beispielsweise 1-Pentyl-imidazol, vorzunehmen, wobei sich ein Addukt aus Ameisensäure und der betreffenden Base bildet. In zwei getrennten hintereinander ablaufenden Destillationsstufen werden zunächst die übrigen Reaktionspartner von dem Addukt und danach die Ameisensäure von der Base getrennt. Als Nachteil dieses Verfahrens muss die Tatsache angesehen werden, dass die Abspaltung der Ameisensäure aus dem Addukt scharfe Destillationsbedingungen erfordert, unter denen sich sowohl die Ameisensäure als auch die Base bereits zu zersetzen beginnen.

Diesen Nachteil vermeiden die Verfahren gemäss der DE-OS 2 853 991 und gemäss der DE-OS 2 914 671. Bei diesen Verfahren werden aus dem Stoffgemisch, das bei der Hydrolyse des Methylformiats anfällt, zunächst die Komponenten Methanol und Methylformiat über Kopf abdestilliert und das aus Ameisensäure und Wasser bestehende Sumpfprodukt einer Flüssig-flüssig-Extraktion mit einem hauptsächlich Ameisensäure aufnehmenden Extraktionsmittel, zugeführt. Aus der Extraktphase werden anschliessend in zwei getrennten Verfahrensschritten zunächst das Wasser und dann die Ameisensäure abdestilliert, ohne dass nennenswerte Zersetzungsreaktionen auftreten, wie sie bei der Arbeitsweise gemäss DE-OS 2 744 313 beobachtet werden. Im Gegensatz zur DE-OS 2 744 313 arbeiten jedoch die Verfahren gemäss den DE-OS 2 853 991 und DE-OS 2 914 671 mit einer Flüssigphasen-Extraktion, um das Wasser auszuschleusen. Hierbei kommen als Extraktionsmittel Carbonsäuren der allgemeinen Formel I zum Einsatz, wie sie oben definiert sind.

Der Erfindung liegt daher die Aufgabe zugrunde, ein wirtschaftliches Verfahren zur Gewinnung von wasserfreier oder weitgehend wasserfreier Ameisensäure zu entwickeln, das weder mit störenden Zersetzungsreaktionen belastet ist noch eine Flüssig-flüssig-Extraktion zur Ausschleusung des nicht umgesetzten Wassers aus der Methylformiat-Hydrolyse benötigt.

Diese Aufgabe wird bei einem Verfahren zur Gewinnung von wasserfreier oder weitgehend wasserfreier Ameisensäure durch Hydrolyse von Methylformiat erfindungsgemäss dadurch gelöst, dass

a) Methylformiat in Gegenwart eines Carbonsäureamids der Formel I hydrolysiert wird,
b) das bei der Hydrolyse entstehende Hydrolysegemisch in einer 1. Destillationskolonne, die mittels in Längsrichtung wirksamer Trenneinrichtungen in einem Zulauf und einen Entnahmeteil unterteilt ist, in Einzelkomponenten zerlegt wird, wobei
- das Hydrolysegemisch in den Zulaufteil eingeführt wird,
- Methanol und nicht umgesetztes Methylformiat als Kopfprodukt und als Seitenprodukt abgezogen werden,
- Extraktionsmittel und Ameisensäure als wasserfreies oder weitgehend wasserfreies Sumpfprodukt abgezogen wird, und
- Wasser - bevorzugt in flüssiger Form - aus dem Entnahmeteil abgeführt wird, und
c) das Sumpfprodukt der 1. Destillationskolonne in einer 2. Destillationskolonne in Ameisensäure und Extraktionsmittel getrennt wird.

Weitere Merkmale des erfindungsgemässen Verfahrens sind Gegenstand der Unteransprüche.

Herzstück der vorliegenden Erfindung und entscheidend für die Wirtschaftlichkeit des Verfahrens ist die Verwendung der in Längsrichtung unterteilten 1. Destillationskolonne, die es ermöglicht, das nicht umgesetzte Wasser der Methylformiat-Hydrolyse in flüssiger oder dampfförmiger, bevorzugt in flüssiger Form und weitgehend ameisensäurefrei zu entnehmen. Dadurch wird die Kondensationswärme des ausgeschleusten Wassers für die Stofftrennungen in der Destillationskolonne ausgenützt.

Ein weiterer Vorteil des erfindungsgemässen Verfahrens besteht darin, dass das Extraktionsmittel bereits im Reaktionsaustrag vorhanden ist und auf diese Weise die Rückveresterung im Zuge der Methanol/Methylformiat-Abtrennung weitgehend unterdrückt wird. Ein Ausführungsbeispiel der Erfindung ist in der Zeichnung dargestellt und wird im folgenden näher beschrieben. Die Zeichnung zeigt ein schematisches Verfahrensfliessbild des erfindungsgemässen Verfahrens.

Die Versuchsanlage bestand aus einem Reaktor 1 zur Durchführung der Hydrolyse und aus zwei Destillationskolonnen 2 und 3 zur Zerlegung des bei der Reaktion entstehenden Hydrolysegemisches in seine Einzelkomponenten. Als Reaktor diente ein Rührbehälter mit einem Reaktionsvo-

lumen von 0,8 l. Die Reaktionsbedingungen betrugen ca. 150°C und ca. 10 bar. Der Reaktor wurde mit den Frischzuläufen 7, bestehend aus 180 g/h Methylformiat mit zusätzlich 5 g/h Methanol, sowie 55,5 g/h Wasser beschickt. Des weiteren wurden dem Reaktor das Kopfprodukt 5 der 1. Destillationskolonne, bestehend aus 244 g/h Methylformiat und 13 g/h Methanol, das untere Seitenentnahmeprodukt 6 der 1. Destillationskolonne, bestehend aus 72,5 g/h Wasser, 4,5 g/h Ameisensäure 1 g/h Di-n-butylformamid und 6 g/h Methanol, sowie das Sumpfprodukt 4 der 2. Destillationskolonne, bestehend aus 657 g/h Di-n-butylformamid und 1 g/h Ameisensäure zugegeben. Die Verweilzeit im Reaktor betrug ca. 30 Minuten. Das als flüssiges Reaktionsprodukt 8 anfallende Hydrolysegemisch, das aus 254 g/h Methylformiat, 115 g/h Methanol, 135,5 g/h Ameisensäure, 77 g/h Wasser und 658 g/h Di-n-butylformamid bestand, wurde in der Mitte des Zulaufteils der 1. Destillationskolonne eingespeist. Neben den bereits genannten Mengen für Kopfprodukt und Seitenentnahmeprodukt 6 der 1. Destillationskolonne wurden aus derselben als Sumpfprodukt 9 125,5 g/h Ameisensäure, 657 g/h Di-n-butylformamid und 6,5 g/h Wasser abgezogen und der 2. Destillationskolonne als Zulaufprodukt zugeführt. Als weiteres Seitenentnahmeprodukt 10 der 1. Destillationskolonne wurden 92 g/h Methanol und 17,5 g/h Methylformiat in flüssiger Form entnommen. Aus der 2. Destillationskolonne wurde neben dem oben genannten Sumpfprodukt 4 als Kopfprodukt 11 das gewünschte Endprodukt in einer Menge von 124,5 g/h Ameisensäure und 6,5 g/h Wasser gewonnen, wobei der Di-n-butylformamidgehalt unter 5 ppm lag.

Die 1. Destillationskolonne – betrieben bei Normaldruck – mit einem Innendurchmesser von 50 mm hatte eine Füllkörperschüttung von 4.000 mm Schütthöhe, bestehend aus Glasraschigringen mit 5 mm Durchmesser. Die Schütthöhe entsprach einer Trennstufenzahl von 50 theoretischen Böden. Zwischen dem 5. und 20. theoretischen Boden war die Destillationskolonne durch einen Steg aus Glas in 2 Teilstücke jeweils gleichen Querschnitts aufgeteilt, so dass in diesem Bereich der Destillationskolonne eine Quervermischung von Flüssigkeits- und Brüdenströmen verhindert wurde. Die Zulaufstelle des Reaktionsgemisches 8 in die Destillationskolonne lag in der Mitte des Zulaufteils – also in der Höhe des 13. theoretischen Bodens –, genau gegenüberliegend auf gleicher Höhe lag im Entnahmeteil die Entnahmestelle des Seitenentnahmeproduktes 6. Das Seitenentnahmeprodukt 10 wurde in Höhe des 43. theoretischen Bodens abgezogen. Das Temperaturprofil innerhalb der Destillationskolonne war folgendes: Temperatur am Kopf 32°C, am 43. theoretischen Boden 53°C, am 13. theoretischen Boden im Entnahmeteil 96°C und im Sumpf 162°C. Das Rücklaufverhältnis betrug 1,0. Am oberen Ende der Längsunterteilung wurde die Flüssigkeit im Vehältnis von 7,3:1 auf den Entnahme- und den Zulaufteil aufgeteilt.

Die 2. Destillationskolonne wurde bei einem Kopfdruck von 80 mbar und einem Rücklaufverhältnis von 1,6 betrieben und wies eine Trennstufenzahl von 20 theoretischen Böden auf. Die Temperaturen am Kopf bzw. im Sumpf der Destillationskolonne betrugen 37°C bzw. 159°C.

Zur Verbesserung der Trennleistung der 1. Destillationskolonne war es von Vorteil, oberhalb der Zulaufstelle – etwa 3 theoretische Böden höher – und unterhalb der Seitenentnahmestelle im längsunterteilten Abschnitt der Destillationskolonne – etwa 3 theoretische Böden tiefer – jeweils 100 g/h Di-n-butylformamid zuzufahren. (Diese Mengen sind in der obengenannten Mengenbilanz nicht berücksichtigt.)

Eine weitere apparative Ausführungsform des erfindungsgemässen Verfahrens besteht darin, dass anstelle einer Destillationskolonne, die im mittleren Teil eine in Längsrichtung wirksame Trenneinrichtung aufweist, ein Kolonnensystem zur Anwendung kommt, bei dem der Zulauf- und der Entnahmeteile als nebeneinander angeordnete Destillationskolonne ausgebildet sind. Ebenso ist es möglich, dass der Entnahmeteil als Verstärkungs- oder als Abtriebskolonne ausgebildet ist.

## Patentansprüche

1. Verfahren zur Gewinnung von wasserfreier oder weitgehend wasserfreier Ameisensäure durch Hydrolyse von Methylformiat unter Einsatz von Extraktionsmitteln, bestehend aus Carbonsäureamiden der allgemeinen Formel I

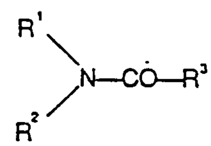

(I)

wobei $R^1$ und $R^2$ Alkylgruppen, die auch zu einem 5- oder 6-gliedrigen Ring verbunden sein können, oder $R^1$ Wasserstoff und $R^3$ Wasserstoff oder einen $C_1$-$C_4$-Alkylrest bedeuten, und die Summe der C-Atome in den Resten $R^1$-$R^3$ 1 bis 14 beträgt, dadurch gekennzeichnet, dass

a) Methylformiat in Gegenwart eines Carbonsäureamids der Formel I hydrolysiert wird,

b) das bei der Hydrolyse entstehende Hydrolysegemisch in einer 1. Destillationskolonne (2), die mittels in Längsrichtung wirksamer Trenneinrichtungen in einen Zulauf- und einen Entnahmeteil unterteilt ist, in Einzelkomponenten zerlegt wird, wobei

- das Hydrolysegemisch (8) in den Zulaufteil eingeführt wird,
- Methanol und nicht umgesetztes Methylformiat als Kopfprodukt (5) und als Seitenprodukt (10) abgezogen werden,
- Extraktionsmittel und Ameisensäure als wasserfreies oder weitgehend wasserfreies Sumpfprodukt (9) abgezogen wird und
- Wasser (6) – bevorzugt in flüssiger Form – aus dem Entnahmeteil abgeführt wird und

c) das Sumpfprodukt (9) der 1. Destillationskolonne in einer 2. Destillationskolonne (3) in Ameisensäure (11) und Extraktionsmittel (4) getrennt wird.

2. Verfahren nach Anspruch 1, dadurch gekennzeichnet, daß als Carbonsäureamide der Formel I Formamide verwendet werden, die mit Ameisensäure keine azeotrop siedenden Mischungen bilden.

3. Verfahren nach Anspruch 1, dadurch gekennzeichnet, dass als Carbonsäureamide N,N-Di-n-propylformamid und/oder N,N-Di-n-butylformamid verwendet werden.

4. Verfahren nach Ansprüchen 1-3, dadurch gekennzeichnet, dass eine zusätzliche Menge des Carbonsäureamids oberhalb der Zulaufstelle des Hydrolysegemisches und unterhalb der Entnahmestelle des Wassers in die 1. Destillationskolonne zugefahren wird.

5. Verfahren nach Ansprüchen 1-4, dadurch gekennzeichnet, dass Zulauf- und Entnahmeteil der 1. Destillationskolonne aus 2 getrennt nebeneinander angeordneten Destillationskolonnen bestehen.

6. Verfahren nach Anspruch 5, dadurch gekennzeichnet, dass der separate Entnahmeteil nur aus einer Verstärkungskolonne besteht, an deren oberen Ende das zurückzuführende Wasser in einem Wärmetauscher kondensiert und in flüssiger Form entnommen wird und die dabei freiwerdende Kondensationswärme zur Verdampfung von Flüssigkeit aus der Hauptkolonne eingesetzt wird.

7. Verfahren nach Anspruch 5, dadurch gekennzeichnet, dass der separate Entnahmeteil nur aus einer Abtriebskolonne besteht, deren unteres Ende mit Brüden aus der Hauptkolonne beheizt wird und aus der das zurückzuführende Wasser als Sumpfprodukt flüssig entnommen wird.

## Claims

1. A process for obtaining anhydrous or substantially anhydrous formic acid by hydrolysis of methyl formate using extracting agents consisting of carboxamides of the formula (I)

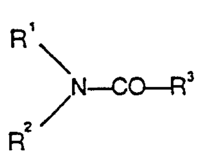

(I)

where $R^1$ and $R^2$ are each alkyl, which may furthermore be joined together to form a 5-membered or 6-membered ring, or $R^1$ is hydrogen, and $R^3$ is hydrogen or $C_1$-$C_4$-alkyl and the sum of the carbon atoms in the radicals $R^1$, $R^2$ and $R^3$ is from 1 to 14, wherein

a) methyl formate is hydrolyzed in the presence of a carboxamide of the formula I

b) the mixture formed during the hydrolysis is separated into its individual components in a 1st distillation column (2) which is divided into a feed part and a take-off part by separating means which are effective in the longitudinal direction,
- the hydrolysis mixture (8) being fed into the feed part,
- methanol and unreacted methyl formate being taken off as a top product (5) and as a side product (10),
- extracting agent and formic acid being taken off as an anhydrous or substantially anhydrous bottom product (9), and
- water (6), preferably in liquid form, being removed from the take-off part, and

c) the bottom product (9) from the 1st distillation column is separated, in a 2nd distillation column (3), into formic acid (11) and the extracting agent (4).

2. A process as claimed in claim 1, wherein a formamide which does not form an azeotropic mixture with formic acid is used as the carboxamide of the formula I.

3. A process as claimed in claim 1, wherein the carboxamides used are N,N-di-n-propylformamide and/or N,N-di-n-butylformamide.

4. A process as claimed in claims 1 to 3, wherein an additional amount of the carboxamide is fed into the 1st distillation column, above the feed point for the hydrolysis mixture and below the take-off point for the water.

5. A process as claimed in claims 1 to 4, wherein the feed part and the take-off part of the 1st distillation column consist of 2 distillation columns arranged separately side by side each other.

6. A process as claimed in claim 5, wherein the separate take-off part consists only of a rectifying column at whose upper end the water to be recycled is condensed in a heat exchanger and removed in liquid form, and the heat of condensation evolved is used for vaporizing liquid from the main column.

7. A process as claimed in claim 5, wherein the separate take-off part consists only of a stripping column whose lower end is heated with vapors from the main column, and from which the water to be recycled is taken off as a bottom product in liquid form.

## Revendications

1. Procédé d'obtention d'acide formique anhydre ou largement anhydre par hydrolyse de formate de méthyle, avec emploi d'agents d'extraction, constitués d'amides d'acide carboxylique de formule générale I

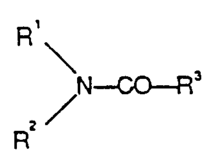

(I)

$R^1$ et $R^2$ représentant des groupes alkyle, qui peuvent aussi être reliés à un noyau à 5 ou 6 chaînes, ou $R^1$ représentait hydrogène et $R^3$ hydrogène ou un reste alkyle en $C_1$-$C_4$, et la somme des atomes C dans les restes $R^1$-$R^3$ étant de 1 à 14, caractérisé par le fait que

a) on hydrolyse du formate de méthyle d'un amide carboxylique de formule I,
b) dans une première colonne de distillation (2) qui est subdivisée, par des moyens de séparation agissant en direction longitudinale, en une partie d'introduction et une partie de prélèvement, on décompose en composants individuels le mélange obtenu par l'hydrolyse,
- le mélange d'hydrolyse (8) étant introduit dans la partie d'introduction,
- le méthanol et le formate de méthyle non entré en réaction étant prélevés comme produit de tête (5) et comme produit annexe (10)
- l'agent d'extraction et l'acide formique étant prélevés comme produit de fond (9) anhydre ou largement anhydre, et,
- l'eau (6) - de préférence sous forme liquide - étant évacuée de la partie de prélèvement et

c) le produit de fond (9) de la première colonne de distillation étant séparé dans une deuxième colonne de distillation (3) en acide formique (11) et agent d'extraction (4).

2. Procédé selon la revendication 1, caractérisé par le fait que, comme amides carboxyliques de formule I, on utilise des formamides qui, avec l'acide formique, ne forment pas de mélanges d'ébullition azéotropes.

3. Procédé selon la revendication 1, caractérisé par le fait que, comme amides carboxyliques, on utilise du N,N-Di-n-propylformamide et/ou N,N-Di-n-butylformamide.

4. Procédé selon la revendication 1 à 3, caractérisé par le fait qu'on amène, dans la première colonne de distillation, une qualité supplémentaire d'amide carboxylique au-dessus de la zone d'introduction du mélange d'hydrolyse et au-dessous de la zone de prélèvement de l'eau.

5. Procédé selon les revendications 1 à 4, caractérisé par le fait que les parties d'introduction et de prélèvement de la première colonne de distillation sont constituées par deux colonnes de distillation déparées, disposées côte à côte.

6. Procédé selon la revendication 5, caractérisé par le fait que la partie de prélèvement séparée n'est constituée que par une colonne de renforcement, à l'extrémité supérieure de laquelle l'eau à réintroduire est condensée dans un échangeur de chaleur et prélevée sous forme liquide et la chaleur de condensation qui y est libérée est utilisée pour vaporiser du liquide à la sortie de la colonne principale.

7. Procédé selon la revendication 5, caractérisé par le fait que la partie de prélèvement séparée n'est constituée que par une colonne d'entraînement dont l'extrémité inférieure est chauffée par des vapeurs provenant de la colonne principale et d'où est prélevée, liquide, comme produit de fond, l'eau à réintroduire.

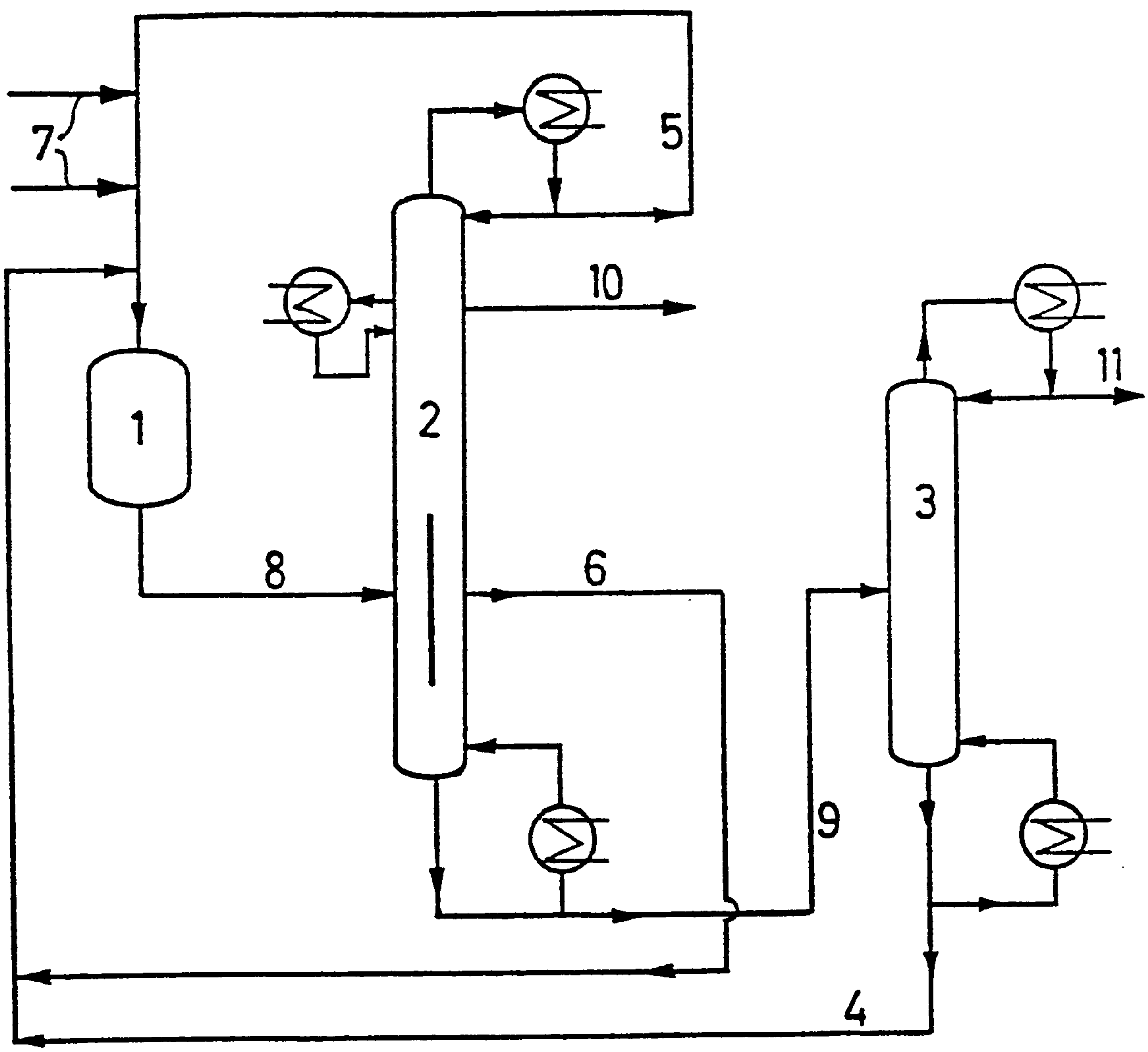
7
5
10
1
2
11
3
8
6
9
4